(19) Europäisches Patentamt
European Patent Office
Office européen des brevets

(11) Veröffentlichungsnummer: **0 161 211**
**B1**

(12) **EUROPÄISCHE PATENTSCHRIFT**

(45) Veröffentlichungstag der Patentschrift: **01.08.90**

(21) Anmeldenummer: **85810154.6**

(22) Anmeldetag: **03.04.85**

(51) Int. Cl.$^5$: **C 07 D 405/12,**
C 07 D 409/12,
C 07 D 403/12,
C 07 D 401/12, A 01 N 47/36

(54) **N-Heterocyclosulfonyl-N'-pyrimidinyl- und -triazinylharnstoffe.**

(30) Priorität: **11.04.84 CH 1822/84**

(43) Veröffentlichungstag der Anmeldung:
**13.11.85 Patentblatt 85/46**

(45) Bekanntmachung des Hinweises auf die Patenterteilung:
**01.08.90 Patentblatt 90/31**

(84) Benannte Vertragsstaaten:
**AT BE CH DE FR GB IT LI NL SE**

(56) Entgegenhaltungen:
EP-A-0 030 142
EP-A-0 039 239
EP-A-0 041 404
EP-A-0 045 196
EP-A-0 057 546
EP-A-0 064 804
EP-A-0 070 698
EP-A-0 071 441
EP-A-0 096 593
EP-A-0 103 543
CH-A- 580 608
DE-A-3 105 453
DE-A-3 225 471

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Schurter, Rolf, Dr.**
**Holzmattstrasse 45**
**CH-4102 Binningen (CH)**
Erfinder: **Meyer, Willy**
**Talweg 49**
**CH-4125 Riehen (CH)**
Erfinder: **Föry, Werner, Dr.**
**Benkenstrasse 65**
**CH-4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 161 211 B1

(56) References cited:

CHEMICAL ABSTRACTS, Band 99, Nr. 7, 15. August 1983, Columbus, Ohio, USA MITSUI TOATSU CHEMICALS INC. "Sulfonylureide derivatives" Seite 563, Spalte 2, Zusammenfassung-Nr. 53 793m

**Beschreibung**

Die vorliegende Erfindung betrifft neue, herbizid wirksame und pflanzenwuchsregulierende Sulfonylharnstoffe, Verfahren zu ihrer Herstellung, die sie als Wirkstoffe enthaltenden Mittel sowie deren Verwendung zum Bekämpfen von Unkräutern, vor allem selektiv in Nutzpflanzenkulturen oder zum Regulieren und Hemmen des Pflanzenwachstums. Ferner betrifft die Erfindung auch neue, als Zwischenprodukte hergestellte Sulfonamide und Derivate davon.

Die erfindungs gemässen Sulfonylharnstoffe entsprechen der Formel I

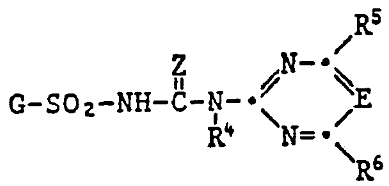 (I)

worin

E Stickstoff oder die Methinbrücke,

Z Sauerstoff oder Schwefel,

$R^4$ Wasserstoff oder $C_1$—$C_4$-Alkyl,

$R^5$ und $R^6$ unabhängig voneinander Wasserstoff, Halogen, $C_1$—$C_4$-Alkyl, $C_1$—$C_4$-Halogenalkyl, $C_4$—$C_4$-Alkoxy, $C_1$—$C_4$-Halogenalkoxy, $C_1$—$C_4$-Alkylthio, $C_3$—$C_6$-Dialkoxyalkyl, $C_1$—$C_4$-Halogenalkylthio, $C_2$—$C_4$-Alkoxyalkyl, $C_3$—$C_6$-Cycloalkyl oder —$NR^{12}R^{13}$,

G eine Gruppe

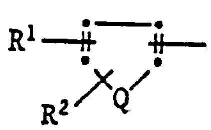 , oder 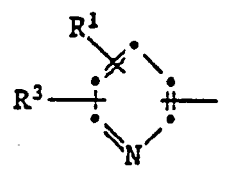 ,

$R^1$ Wasserstoff, Halogen, Nitro, $C_1$—$C_4$-Alkyl, $C_1$—$C_4$-Alkoxy, Trifluoromethyl, Trifluoromethoxy, Difluoromethoxy, $C_1$—$C_4$-Alkylcarbonyl oder —$COOR^{14}$,

$R^2$ eine Gruppe

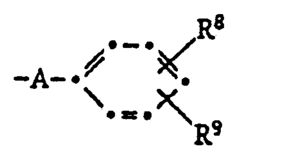 ,

$R^3$ eine Gruppe

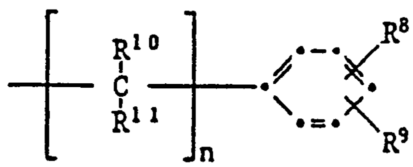 ,

Q Schwefel oder,

A Sauerstoff, Schwefel, —SO—, —$SO_2$— oder —$(CR^{10}R^{11})_m$— m und n die Zahlen null, eins oder zwei,

$R^8$ Wasserstoff, $C_1$—$C_4$-Alkyl, $C_1$—$C_4$-Alkoxy, Halogen, $C_1$—$C_4$-Halogenalkyl, Nitro, —$COOR^{14}$-Halogenalkoxy, —O—$CR^{15}R^{16}$—$COOR^{14}$ oder —O—$CR^{15}R^{16}$—CN,

$R^9$ Wasserstoff, Halogen, $C_1$—$C_4$-Alkyl, $C_1$—$C_4$-Alkoxy, $C_2$—$C_4$-Alkoxyalkyl oder $C_2$—$C_4$-Alkoxyalkoxy,

$R^{10}$ und $R^{11}$ unabhängig voneinander Wasserstoff oder $C_1$—$C_4$-Alkyl,

$R^{12}$ und $R^{13}$ unabhängig voneinander Wasserstoff oder $C_1$—$C_4$-Alkyl,

$R^{14}$ Wasserstoff, $C_1$—$C_4$-Alkyl, $C_2$—$C_4$-Alkenyl, $C_3$—$C_4$-Alkinyl oder durch $C_1$—$C_4$-Alkoxy, Halogen oder Phenyl substituiertes $C_1$—$C_4$-Alkyl und

$R^{15}$ und $R^{16}$ unabhängig voneinander Wasserstoff oder $C_1$—$C_4$-Alkyl bedeuten, sowie den Salzen dieser Verbindungen.

Harnstoffverbindungen, Triazinverbindungen und Pyrimidinverbindungen mit herbizider Wirkung sind allgemein bekannt. Herbizide und pflanzenwuchsregulierende Sulfonylharnstoffverbindungen, welche als Strukturmerkmale an die Sulfonylgruppe gebundene heterocyclische aromatische Ringe aufweisen, sind in der Literatur beschrieben. So finden sich Pyrrolysulfonylharnstoffe in der EP—A—39239, Thienyl- und Furylsulfonylharnstoffe in EP—A—30142, EP—A—41404, EP—A—57546 und EP—A—64804, Pyridinylsulfonylharnstoffe in EP—A—57546, Benzofuryl- und -thienylsulfonylharnstoffe in EP—A—45196

und Indolylsulfonylharnstoffe in EP—A—70698. Phenoxypyridinylsulfonylharnstoffe wurden kürzlich aus der EP—A—103543 bekannt.

In den Definitionen ist unter Alkyl geradkettiges oder verzweigtes Alkyl zu verstehen; z.B.: Methyl, Aethyl, n-Propyl, i-Propyl oder die vier isomeren Butyl.

Unter Alkoxy ist zu verstehen: Methoxy, Aethoxy, n-Propyloxy, i-Propyloxy oder die vier isomeren Butyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio.

Cycloalkyl steht im allgemeinen für Cyclopropyl, Cyclobutyl, Cyclopentyl oder Cyclohexyl.

Unter Halogen selbst sowie als Teil eines Substituenten wie in Halogenalkoxy, Halogenalkylthio oder Halogenalkyl sind Fluor, Chlor und Brom, vorzugsweise jedoch Fluor und Chlor zu verstehen. Halogenalkyl selbst oder als Teil von Halogenalkoxy oder Halogenalkylthio steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Beispiele für Alkoxyalkyl sind: Methoxymethyl, Methoxyäthyl, Methoxypropyl, Aethoxyäthyl, Aethoxymethyl oder Propyloxymethyl. Beispiele für Alkoxyalkoxy sind: Methoxymethoxy, Methoxyäthoxy, Methoxypropyloxy, Aethoxymethoxy, Aethoxyäthoxy sowie Propyloxymethoxy. Unter Dialkoxyalkyl sollen im Rahmen der vorliegenden Erfindung im allgemeinen folgende Reste verstanden werden: Dimethoxymethyl, 2,2-Dimethoxyäthyl, 1,2-Dimethoxyäthyl, 1,2-Diäthoxyäthyl, 2,2-Diäthoxyäthyl, 2,2-Dimethoxypropyl, 3,3-Dimethoxypropyl, 1,1-Dimethoxyäthyl, 1,1-Diäthoxyäthyl, 1,1-Dimethoxypropyl, 2,3-Dimethoxypropyl, 1,1-Dimethoxybutyl, 2,2-Dimethoxybutyl, 3,3-Dimethoxybutyl, 4,4-Dimethoxybutyl, vorzugsweise aber die geminalen Dialoxyalkylreste, die auch als Acetale charakterisiert werden können.

Alkenylreste sind Vinyl, Allyl, 2-Butenyl, 3-Butenyl oder Methallyl. Alkinylreste sind Propargyl, 2-Butinyl oder 3-Butinyl. Bevorzugt sind Allyl und Propargyl.

Unter Alkylcarbonylresten sollen insbesondere verstanden werden: Acetyl, Propionyl, Butyryl, Isobutyryl, Valeryl, Isovaleryl oder Pivaloyl.

Die unter G definierten aromatischen Heterocyclensysteme umfassen beispielsweise folgende Grundkörper: Pyridin, oder Thiophen.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Träthylamin, Tripropylamin, Chinuclidin, Pyridin, Chinolin und i-Chinolin, insbesondere aber Aethyl-, Propyl-, Diäthyl- oder Triäthylamin, vin allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Tetraäthylammoniumkation, das Trimethyläthylammoniumkation, aber auch das Ammoniumkation.

Unter den Verbindungen der Formel I sind diejenigen bevorzugt, in denen entweder.

a) Z Sauerstoff ist oder

b) $R^1$ für Wasserstoff, Cl, F, $CH_3$, $C_2H_5$, $CH_3O$, $CF_3$, $CH_3CO$ steht oder

c) $R^5$ und $R^6$ zusammen höchstens 4 Kohlenstoffatome enthalten und $R^4$ für Wasserstoff steht oder

d) G für 3-Phenylpyridin-2-yl steht oder

e) G für gegebenenfalls mit Cl, $CH_3$ oder $CH_3O$ substituiertes 3-Phenylthiophen-2-yl, 3-Phenoxythiophen-3-yl oder 2-Phenylthiophen-3-yl steht oder

f) G für 2-$C_1$—$C_4$-Alkoxycarbonyl-4-phenylthiophen-3-yl oder 2-$C_1$—$C_4$-Alkoxycarbonyl-5-phenylthiophen-3-yl steht.

Als weitere hervorzuhebende Untergruppen von Verbindungen der Formel I sind diejenigen zu nennen, in denen entweder

aa) Z Sauerstoff, $R^4$ Wasserstoff und G 3-Phenylpyridin-2-yl bedeuten und $R^5$ und $R^6$ zusammen höchstens 4 Kohlenstoffatome enthalten oder

bb) Z Sauerstoff, $R^4$ Wasserstoff und G 3-Phenylthiophen-2-yl, 3-Phenoxythiophen-2-yl oder 2-Phenylthiophen-3-yl bedeuten und $R^5$ und $R^6$ zusammen höchstens 4 Kohlenstoffatome enthalten.

cc) Z Sauerstoff, $R^4$ Wasserstoff und G 2-$C_1$—$C_4$-Alkoxycarbonyl-4-phenylthiophen-3-yl oder 2-$C_1$—$C_4$-Alkoxycarbonyl-5-phenylthiophen-3-yl bedeuten und $R^5$ und $R^6$ zusammen höchstens 4 Kohlenstoffatome enthalten.

Als bevorzugte Einzelverbindung der Formel I ist zu nennen:

N-(2-Methoxycarbonyl-4-phenylthiophen-3-ylsulfonyl)-N'-(4-methoxy-6-methylpyrimidin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein Substituiertes Sulfonamid der Formel II

$$G{-}SO_2{-}NH_2 \qquad \text{(II)},$$

worin G die unter Formel I gegebene Bedeutung hat, in Gegenwart einer Base mit einem Carbamat oder Thiocarbamat der Formel III

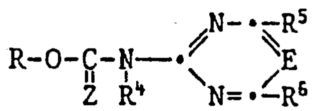

(III)

worin E, $R^4$, $R^5$, $R^6$ und Z die unter Formel I gegebene Bedeutung haben und R für Phenyl, Alkyl oder substituiertes Phenyl steht, umsetz.

Nach einem zweiten Verfahren gelangt man zu Verbindungen der Formel I, indem man ein Sulfonylcarbamat oder Sulfonylthiocarbamat der Formel IV

$$G{-}SO_2{-}NH{-}\underset{\underset{\displaystyle Z}{\|}}{C}{-}O{-}R \qquad \text{(IV)},$$

worin G und Z die unter Formel I gegebene Bedeutung haben und R für Phenyl, alkyl oder substituiertes Phenyl steht, mit einem Amin der Formel V

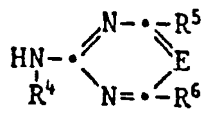

(V)

worin E, $R^4$, $R^5$ und $R^6$ die unter Formel I gegebene Bedeutung haben, umsetzt.

Schliesselich kann man die Verbindungen der Formel I auch erhalten, indem man ein Sulfonylisocyanat oder Sulfonylisothiocyanat, der formel VI

$$G{-}SO_2{-}N{=}C{=}Z \qquad \text{(VI)},$$

worin G und Z die unter Formel I gegebenen Bedeutungen haben, mit einem Amin der oben angegebenen Formel V umsetzt.

Die erhaltenen Harstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril, oder Propionitril, Amide wie Dimethylformamid, Diäthylformamid oder N-Methylpyrrolidinon. Die Reaktionstemperaturen Liegen vorzugsweise zwischen $-20°$ und $+120°C$. Die Umsetzungen der Kupplungsverfahren verlaufen im allgemeinen leicht exotherm und können bei Raumtemperatur durchgeführt werden. Zwecks Abkürzung der Reaktionszeit oder auch sum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo[2.2.2]-octan, 1,5-Diazabicyclo[4.3.0]non-5-en oder 1,8-Diazabicyclo[5.4.0]undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Zwischenprodukte der Formeln, II, IV, und VI sind neu. Diese neuen Verbindungen wurden speziell für die Synthese der Verbindungen der Formel I entwickelt. Sie bilden daher einen weiteren Askpekt der verliegenden Erfindung.

Die Zwischenprodukte der Formel II werden nach an sich bekannten Verfahren hergestellt. So erhält man die Verbindungen der Formel II beispielsweise, indem man ein Amin der Formel VII

$$G—NH_2 \quad (VII),$$

worin G die unter Formel I gegebenen Bedeutung hat, in Salzsäure diazotiert und die Diazogruppe mit Schwefeldioxid in Gegenwart eines Katalysators wie Kupferchlorid umsetzt und das entstandene Sulfonylchlorid der Formel VIII

$$G—SO_2—Cl \quad (VIII),$$

worin G die unter Formel I angegebene Bedeutung hat, mit Ammoniak umsetzt. Die als Ausgangsprodukte verwendeten entsprechenden Amine sind bekannt oder nach bekannten Methoden, beispielsweise durch Reduktion aus den entsprechenden Nitroverbindungen herstellbar.

Weiter kann man die Verbindungen der Formel II erhalten, indem man eine Sulfonsäure der Formel IX

$$G—SO_2—OH \quad (IX)$$

worin G die unter Formel I gegebenen Bedeutung hat, durch Behandeln mit einem Chlorierungsmittel wie $PCl_5$ $POCl_3$, $COCl_2$ oder $SOCl_2$ in das entsprechende Sulfonylchlorid der Formel VIII überführt und dieses mit Ammoniak umsetzt.

Ebenso kann man die Verbindungen der Formel II erhalten, indem man einen Benzylthioäther der Formel X

$$G—S—CH_2—C_6H_5 \quad (X)$$

worin G die unter Formel I gegebenen Bedeutung hat, mit Chlor behandelt und das entstandene Sulfonylchlorid der Formel VIII mit Ammoniak umsetzt.

In einigen Fällen erhält man die Sulfonylchloride der Formel VIII durch direkte Sulfochlorierung der substituierten Verbindung der Formel XI

$$G—H \quad (XI)$$

worin G die unter Formel I gegebenen Bedeutung hat, mit Chlorsulfonsäure $ClSO_3H$.

Die sulfonylisocyanate der Formel VI können beispielsweise durch Umsetzungen der sulfonamide der Formel II mit Phosgen in Anwesenheit von Butylisocyanat in einem inerten Lösungsmittel, bei Rückflusstemperatur erhalten werden. Aehnliche Darstellungen sind im "Neuere Methoden der Päparativen organischen Chemie", Band VI, 211—229, Verlag Chemie, Weinheim, 1970, beschrieben.

Die Sulfonylisothiocyanate der Formel VI werden durch Behandlung der Sulfonamide der Formel II mit Schwefelkohlenstoff und Kaliumhydroxid und anschliessende Umsetzung des Dikaliumsalzes mit Phosgen erhalten. Solche Verfahren sind in Arch. Pharm. *299*, 174 (1966) beschrieben.

Die Sulfonylcarbambate der Fomel IV werden durch Umsetzung der Sulfonamide der Formel II mit einem Kohlensäureester in Gegenwart einer Base erhalten. Aehnliche Verfahren sind in der japanischen Patentschrift 61 169 erwähnt.

Die als Ausgangsmaterialien verwendeten Aminiopyrimidine und -triazine der Formel V sowie entsprechende Carbamate der Formel III sind entweder bekannt oder sie lassen sich nach bekannten Methoden aus Literatur beschriebenen Verbindungen erhalten.

Die Verbindungen der Formeln VII, VIII, IX, X und XI sind bekannt oder können analog zu bekannten Verfahren hergestellt werden.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektivwuchschemmende und selektiv-herbizide Eingenschaften aus, die sie Ausgezeichnet sum Einsatz in Kulturen von Nutzpflanzen, insbesondere in Getreide, Baumwolle, Soja und Mais und ganz besonders Reis, befähigen. Es werden dabei teilweise auch Unkräuter geschädigt, welchen bischer nur mit Totalherbiziden beizukomnen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. in der Landwirtschaft in tropischen Gegenden häufig als "cover crops" (Bodenbedecker) angepflanzte Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen an Wirkstoffen der Formel I werden alle getesteten Pflanzen in ihrere Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eingenschaften Können auch hochdisperse Kieselsäure oder hochdisperse saugfähige Polymerisate zugesetzt werden. Als gekörnte, adsorbptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter tensiden sind auch Tensidgemische zu verstehen.

Geeignete anionische Tenside können sowohl sog. wasserlösliche Seifen als auch wasserlösliche synthetische oberflächenaktive Verbunden sein.

Als Seifen seien die Alkali-, Erdalkali- oder gegebenenfalls substituierte Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschleisst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside komnen in erster Linie Polyglykolätherderivate von aliphatischen oder cycloaliphatischen Alkoholen, gesättigten oder ungesättigten Fettsäuren und Alkylphenolen in Frage, die 34

bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindugen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tenhiden handelt es sich vor allem un quaternäre Ammoniumsalze, welche als N-Substituenten mindestens einem Alkylrest mit 8 bis 22 C-Atomen enthalten und als weitere Substituenten niederige, gegebenenfalls halogenierte Alkyl-, Benzoyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formelierungstecknik gebräuchlichen Tenside sind u.a. in folgenden Publikatinen beschrieben:

"Mc Cutcheon's Detergents and Emulsifiers Annual" MC Publishing Corp., Ridgewood, New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I—III, Chemical Publishing Co., New York, 1980—1981.

Die pestiziden zubereitungen enthalten in der Regel 0,1 bis 95%, insbesondere 0,1 bis 80% Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent).

| | |
|---|---|
| Emulgierbare Konzentrate: | |
| Aktiver Wirkstoff: | 1 bis 20%, bevorzugt 5 bis 10% |
| oberflächenaktive Mittel: | 5 bis 30%, vorzugsweise 10 bis 20% |
| flüssiges Trägermittel: | 50 bis 94%, vorzugsweise 70 bis 85% |
| Stäube: | |
| Aktiver Wirkstoff: | 0,1 bis 10% vorzugsweise 0,1 bis 1% |
| festes Trägermittel: | 99,9 bis 90%, vorzugsweise 99,9 bis 99% |
| Suspensions-Konzentrate: | |
| Aktiver Wirkstoff: | 5 bis 75%, vorzugsweise 10 bis 50% |
| Wasser: | 94 bis 24%, vorzugsweise 88 bis 30% |
| oberflächenaktives Mittel: | 1 bis 40%, vorzugsweise 2 bis 30% |
| Benetzbares Pulver: | |
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90% |
| Granulate: | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

Beispiel H1: N-(2-Methoxycarbonyl-4-phenylthiophen-3-yl-sulfonyl)-N'-(A,6-dimethoxypyrimidin-2-yl)-harnstoff (Verbindung Nr. 3.09).

a) 2-Methoxycarbonyl-4-phenylthiopen-3-ylsulfonylchlorid.

10,9 g (0.04 mol) 3-Amino-2-methoxycarbonyl-4-phenylthiophen werden in 20 ml konz. Salzsäure gelöst und bei −5C° mit 3,65 g Natrium nitrit in 10 ml Wasser diazotiert. 30 Minuten nach dem zutropfen wird die Diazoniumsalzlösung portionsweise zu einem Gemisch von 20 ml Dichloräthan, 0,45 g Benzyltriäthylammoniumchlorid, 0,3 g Kupfer-(I)-chlorid, 0,3 g Kupfer-(II)-chlorid und 6 g Schwefeldioxid gegeben. Nach Beendigung der Zugabe bei 5 bis 10°C wird das Reaktionsgemisch eine Stunde bei Raumtemperatur gerührt und anschliessend dreimal mit Dichloräthan extrahiert. Die vereinigten Extrakte werden mit Aktivkohle behandelt, filtriert und eingeengt. Das erhaltene 2-Methoxycarbonyl-4-phenylthiophen-3-ylsulfonylchlorid wird ohne Reinigung im folgenden Rekatinsschritt weiterverwendet.

b) 2-Methoxycarbonyl-4-phenylthiophen-3-ylsulfonamid Das unter a) erhaltene 2-Methoxycarbonyl-4-phenylthiophen-3-yl-sulfonylchlorid wird in 120 ml Tetrahydrofuran gelöst. Dann werden bei −10°C 2,2 g Ammoniak-Gas eingeleitet. Anschliessend wird das Reaktionsgemisch bei Raumtemperatur gerührt und nach Beendigung der Reaktion eingeengt. Der Rückstand wird durch Chromatographie an einem Kieselgel gereinigt. Das Lösungsmittel (Essigsäureäthylester/Cyclohexan 2:1) wird abdestilliert und man erhält 5,2 g 2-Methoxycarbonyl-4-phenylthiophen-3-ylsulfonamid vom Smp. 172—175°C.

c) 2,4 g (0,008 mol) 2-Methoxycarbonyl-4-phenylthiophen-3-ylsulfonamid, 2,2 g N-(4,6-Dimethoxypyrimidin-2-yl)-phenylcarbamat, 1,2 g 1,8-Diazabicyclo[5.4.0]undec-7-en und 30 ml Acetonitril werden 16 Stunden bei Raumtemperatur gerührt. Dann wird das Reaktionsgemisch auf ein Eis-Wassergemisch gegossen und mit Methansulfonsäure angesäuert. Der erhaltene Niederschlag wird filtriert, mit Wasser gewaschen und getrocknet. Man erhält so 3,4 g (95%) N-(2-Methoxycarbonyl-4-phenylthiophen-3-ylsulfonyl)-N'-(4,6-dimethoxy-pyrimidin-2-yl)-harnstoff, Smp 225—227°C.

In analoger Weise werden die in den angeschlossenen Tabellen aufgelisteten Zwischen- und Endprodukte hergestellt.

Tabelle 1

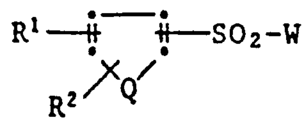

| Verb. Nr. | Q | W | Position —$SO_2$—W | $R^1$ | $R^2$ | Smp. [°C] |
|---|---|---|---|---|---|---|
| 1.01 | S | $NH_2$ | 2 | H | 3-Phenyl | |
| 1.02 | S | $NH_2$ | 2 | 5—$CH_3$ | 3-Phenyl | |
| 1.03 | S | $NH_2$ | 2 | 5—Cl | 3-Phenyl | |
| 1.04 | S | $NH_2$ | 3 | H | 2-Phenyl | |
| 1.05 | S | $NH_2$ | 5 | H | 2-Phenyl | |
| 1.06 | S | $NH_2$ | 3 | H | 2-Benzyl | |
| 1.07 | S | $NH_2$ | 3 | H | 2-Phenoxy | |
| 1.08 | S | —N=C=O | 2 | H | 3-Phenyl | |
| 1.09 | S | —N=C=o | 3 | H | 2-Phenyl | |
| 1.10 | S | $NH_2$ | 3 | 5—$CH_3$ | 2-Phenoxy | |
| 1.11 | S | Cl | 3 | 2—$COOCH_3$ | 4-Phenyl | |
| 1.12 | S | Cl | 3 | 2—$COOC_2H_5$ | 4-Phenyl | |
| 1.13 | S | $NH_2$ | 3 | 2—$COOCH_3$ | 4-Phenyl | 172—175 |
| 1.14 | S | $NH_2$ | 3 | 2—$COOC_2H_5$ | 4-Phenyl | |
| 1.15 | S | Cl | 3 | 2—$COOCH_3$ | 5-Phenyl | |
| 1.16 | S | Cl | 3 | 2—$COOC_2H_5$ | 5-Phenyl | |
| 1.17 | S | $NH_2$ | 3 | 2—$COOCH_3$ | 5-Phenyl | 180 |
| 1.18 | S | $NH_2$ | 3 | 2—$COOC_2H_5$ | 5-Phenyl | 167 |
| 1.19 | S | $NH_2$ | 3 | H | 4-Phenyl | |
| 1.20 | S | Cl | 3 | H | 4-Phenyl | |
| 1.21 | S | Cl | 3 | 2—$CPPCH_3$ | 4-(Fluor-phenyl) | |

Tabelle 1 (Fortsetzung)

| Verb. Nr. | Q | W | Position —$SO_2$—W | $R^1$ | $R^2$ | Smp. [°C] |
|---|---|---|---|---|---|---|
| 1.22 | S | $NH_2$ | 3 | 2—$COOCH_3$ | 4-(2-Fluor-phenyl) | |
| 1.23 | S | Cl | 3 | 2—$COOCH_2H_5$ | 4-(2-Fluor-phenyl) | |
| 1.24 | S | $NH_2$ | 3 | 2—$COOCH_2H_5$ | 4-(2-Fluor-phenyl) | |
| 1.25 | S | Cl | 3 | 2—$COOCH_3$ | 4-(3-Fluor-phenyl) | |
| 1.26 | S | $NH_2$ | 3 | 2—$COOCH_3$ | 4-(3-Fluor-phenyl) | |

Tabelle 2

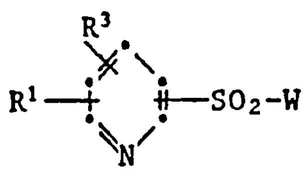

| Verb. Nr. | W | Position —$SO_2$—W | $R^1$ | $R^3$ |
|---|---|---|---|---|
| 2.01 | $NH_2$ | 2 | H | 3-Phenyl |
| 2.02 | $NH_2$ | 2 | H | 3-Benzyl |
| 2.03 | $NH_2$ | 2 | 5—Cl | 3-Phenyl |
| 2.04 | $NH_2$ | 3 | 2—Cl | 6-Phenyl |
| 2.05 | Cl | 3 | H | 3-Phenyl |
| 2.06 | Cl | 3 | 5—Cl | 3-Phenyl |

Tabelle 3

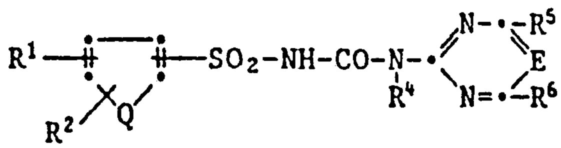

| Verb. Nr. | Q | Position —$SO_2$— | $R^1$ | $R^2$ | $R^4$ | $R^5$ | $R^6$ | E | Smp. [°C] |
|---|---|---|---|---|---|---|---|---|---|
| 3.01 | S | 2 | H | 3-Phenyl | H | $OCH_3$ | $OCH_3$ | CH | |
| 3.02 | S | 2 | H | 3-Phenyl | $CH_3$ | $OCH_3$ | $CH_3$ | CH | |
| 3.03 | S | 2 | 5—$CH_3$ | 3-Phenyl | H | $OCH_3$ | $OCH_3$ | CH | |
| 3.04 | S | 2 | 5—Cl | 3-Phenyl | H | $OCH_3$ | $OCH_3$ | N | |
| 3.05 | S | 2 | H | 3-Phenyl | H | $OCH_3$ | $OCH_3$ | N | |
| 3.06 | S | 3 | H | 2-Phenyl | H | $OCH_3$ | $OCH_3$ | N | |
| 3.07 | S | 3 | H | 2-Benzyl | H | $OCH_3$ | $OCH_3$ | CH | |
| 3.08 | S | 3 | H | 2-Phenoxy | H | $OCH_3$ | $OCH_3$ | CH | |
| 3.09 | S | 3 | 2—$COOCH_3$ | 4-Phenyl | H | $OCH_3$ | $OCH_3$ | CH | 225—227 |
| 3.10 | S | 3 | 2—$COOCH_3$ | 4-Phenyl | H | $OCH_3$ | $CH_3$ | CH | 196 (Zwers.) |
| 3.11 | S | 3 | 2—$COOC_2H_5$ | 5-Phenyl | H | $OCH_3$ | $OCH_3$ | CH | 178—179 |
| 3.12 | S | 3 | 2—$COOC_2H_5$ | 5-Phenyl | H | $OCH_3$ | $CH_3$ | CH | 202 (Zers.) |
| 3.13 | S | 3 | 2—$COOCH_3$ | 5-Phenyl | H | $OCH_3$ | $CH_3$ | CH | 204—206 |
| 3.14 | S | 3 | 2—$COOCH_3$ | 5-Phenyl | H | $OCH_3$ | $OCH_3$ | CH | 188—189 |
| 3.15 | S | 3 | H | 4-Phenyl | H | $OCH_3$ | $OCH_3$ | CH | |
| 3.16 | S | 3 | H | 4-Phenyl | H | $CH_3$ | $OCH_3$ | CH | |
| 3.17 | S | 3 | H | 4-Phenyl | H | $OCH_3$ | $OCH_3$ | N | |
| 3.18 | S | 3 | H | 4-(2-Fluor-phenyl) | H | $OCH_3$ | $OCH_3$ | CH | |
| 3.19 | S | 3 | H | 4-(2-Fluor-phenyl) | H | $OCH_3$ | $OCH_3$ | CH | |

Tabelle 3 (Fortsetzung)

| Verb. Nr. | Q | Position $-SO_2-$ | $R^1$ | $R^2$ | $R^4$ | $R^5$ | $R^6$ | E | Smp. [°C] |
|---|---|---|---|---|---|---|---|---|---|
| 3.20 | S | 3 | H | 4-(2-Fluor-phenyl) | H | $OCH_3$ | $OCH_3$ | N | |
| 3.21 | S | 3 | $2-COOCH_3$ | 4-(2-Fluor-phenyl) | H | $OCH_3$ | $OCH_3$ | CH | |
| 3.22 | S | 3 | $2-COOCH_3$ | 4-(2-Fluor-phenyl) | H | $OCH_3$ | $OCH_3$ | CH | |
| 3.23 | S | 3 | $2-COOC_2H_5$ | 4-(2-Fluor-phenyl) | H | $CH_3$ | $OCH_3$ | CH | |
| 3.24 | S | 3 | $2-COOC_2H_5$ | 4-(2-Fluor-phenyl) | H | $OCH_3$ | $OCH_3$ | CH | |
| 3.25 | S | 3 | 2—Cl | 4-(2-Fluor-phenyl) | H | $OCH_3$ | $OCH_3$ | CH | |
| 3.26 | S | 3 | 2—Cl | 4-(2-Fluor-phenyl) | H | $CH_3$ | $OCH_3$ | CH | |
| 3.27 | S | 3 | 2—Cl | 4-Phenyl | H | $CH_3$ | $OCH_3$ | CH | |
| 3.28 | S | 3 | 2—Cl | 4-Phenyl | H | $OCH_3$ | $OCH_3$ | CH | |
| 3.29 | S | 3 | 2—Cl | 4-Phenyl | H | $OCH_3$ | $OCH_3$ | N | |
| 3.30 | S | 3 | 5—Cl | 4-Phenyl | H | $OCH_3$ | $OCH_3$ | CH | |
| 3.31 | S | 3 | 5—Cl | 4-Phenyl | H | $CH_3$ | $OCH_3$ | CH | |
| 3.32 | S | 3 | $2-COOCH_3$ | 4-(4-Methyl-phenyl) | H | $CH_3$ | $OCH_3$ | CH | |
| 3.33 | S | 3 | $2-COOCH_3$ | 4-(4-Methyl-phenyl) | H | $OCH_3$ | $OCH_3$ | CH | |
| 3.34 | S | 3 | 2—Cl | 5-Phenyl | H | $OCH_3$ | $OCH_3$ | CH | |

Tabelle 4

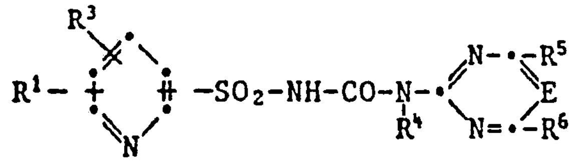

| Verb. Nr. | Position —$SO_2$— | $R^1$ | $R^2$ | $R^4$ | $R^5$ | $R^6$ | E |
|---|---|---|---|---|---|---|---|
| 4.01 | 2 | H | 3-Phenyl | H | $OCH_3$ | $OCH_3$ | CH |
| 4.02 | 2 | H | 3-Phenyl | H | $OCH_3$ | $OCH_3$ | N |
| 4.03 | 2 | H | 3-Phenyl | $CH_3$ | $OCH_3$ | $OCH_3$ | CH |
| 4.04 | 2 | H | 3-Benzyl | H | $OCH_3$ | $OCH_3$ | CH |
| 4.05 | 2 | 5—Cl | 3-Phenyl | H | $OCH_3$ | $OCH_3$ | CH |
| 4.06 | 2 | 5—Cl | 3-Phenyl | H | $OCH_3$ | $OCH_3$ | N |
| 4.07 | 3 | 2—Cl | 6-Phenyl | H | $OCH_3$ | $OCH_3$ | CH |
| 4.08 | 3 | H | 3-Phenyl | H | $OCH_3$ | $OCH_3$ | CH |
| 4.09 | 3 | H | 3-Phenyl | H | $CH_3$ | $OCH_3$ | N |

Formulierungsbeispiel

Beispiel F I: Formulierungsbeispiele für Wirkstoffe der Formel I (% = Gewichtsprozent)

| a) *Spritzpulver* | a) | b) | c) |
|---|---|---|---|
| Wirkstoff | 20% | 50% | 0,5% |
| Na-Ligninsulfonat | 5% | 5% | 5% |
| Na-Laurylsulfat | 3% | — | — |
| Na-Diisobutylnaphthalinsulfonat | — | 6% | 6% |
| Octylphenolpolyäthylenglykol-äther /7—8 Mol AeO) | — | 2% | 2% |
| Hochdisperse Kieselsäure | 5% | 27% | 27% |
| Kaolin | 67% | — | — |
| Natriumchlorid | — | — | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzenration verdünnen lassen.

| b) *Emulsions-Konzentrat* | a) | b) |
|---|---|---|
| Wirkstoff | 10% | 1% |
| Octylphenolpolyäthylenglykoläther (4—5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) *Stäubemittel* | a) | b) |
|---|---|---|
| Wirkstoff | 0,1% | 1% |
| Talkum | 99,9% | — |
| Kaolin | — | 99% |

Man erhält anwendungsfertige Stäubemittel, indem der Wirkstoff mit dem Träger vermischt und auf einer geeigneten Mühle vermahlen wird.

| d) *Extruder Granulat* | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) *Umhüllungs-Granulat* | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) *Suspensions-Konzentrat* | a) | b) |
|---|---|---|
| Wirkstoff | 40% | 5% |
| Aethylenglykol | 10% | 10% |
| Nonylphenolpolyäthylenglykoläther (15 Mol Aeo) | 6% | 1% |
| Na-Ligninsulfonat | 10% | 5% |
| Carboxymethylcellulose | 1% | 1% |
| 37% ige wässrige Formaldehyd-Lösung | 0,2% | 0,2% |
| Silikonöl in Form einer 75 %igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) *Salzlösung* | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel B 1: Herbizidwirkung von dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis und Stellaria media. Die Versuchsgefässe werden anschliessend in einer Klimakammer bei 20°C, einer Beleuchtung von ca. 20 kLux und einer relativen Luftfeuchtigkeit von 70% gehalten. Während einer Keimphase von 4 bis 6 Tagen werden die Töpfe zur Erhöhung der örtlichen Luftfeuchtigkeit mit lichtdurchlässigem Material abgedeckt und mit deionisiertem Waser gegossen. Nach dem 5.Tag wird dem Giesswasser 0,5% eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1: Pflanze nicht gekeimt oder total abgestorben
2—3: sehr stark Wurkung
4—6: mittlere Wirkung
7—8: schwache Wirkung
9: keine Wirkung (wie unbehandelte Kontrolle).

pre-emergente Wirkung:
Konzentration der Wirkstoffemulsion: 70,.8 ppm

| Testpflanze Wirkstoff Nr | Nasturtium | Stellaria | Agrostis | Digitaria |
|---|---|---|---|---|
| 3.09 | 1 | 2 | 1 | 2 |
| 3.10 | 1 | 2 | 1 | 2 |
| 3.11 | 2 | 2 | 2 | 2 |
| 3.12 | 2 | 2 | 3 | 2 |
| 3.14 | 2 | 3 | 3 | 3 |

Beispiel B 2: Wuchshemmung bie tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 600 lux Kunstlicht, pro Tag 14 Stunden, bei Temperaturen von 27° bie Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit dem Wirkstoffen der Formel I behandelten Pflanzen eine deutliche Reduktion des Neuzuwachses (weniger als 20% des Neuzuwachses bei unbehandelten Kontrollpflanzen), ohne dass dabei die Versuchspflanzen geschädigt werden.

Beispiel B 3: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5—6 Trifolia-Blattstadium. Zu diesem Zeitpunkt werden die Pflanzen mit der wässrigen Brühe eines Wirkstoffs der Formel I bis zu guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B 4: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60—90% der Kontrolle), sowie teilweise eine Zunahme der Stengeldurchmesser auf.

Beispiel B 5: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10—30% im Vergleich zur unbehandelten Kontrolle.

**Patentansprüche für die Vertragsstaaten: BE CH DE FR GB IT LI NL SE**

1. Sulfonylharnstoffe der Formel I

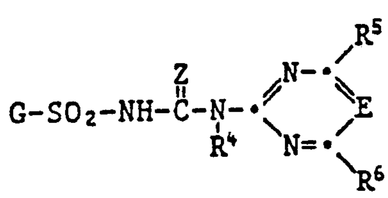

(I)

worin

E Stickstoff oder die Methinbrücke,

Z Sauerstoff oder Schwefel,

$R^4$ Wasserstoff oder $C_1$—$C_4$-Alkyl,

$R^5$ und $R^6$ unabhängig voneinander Wasserstoff, Halogen, $C_1$—$C_4$-Alkyl, $C_1$—$C_4$-Halogenalkyl, $C_4$—$C_4$-Alkoxy, $C_1$—$C_4$-Halogenalkoxy, $C_1$—$C_4$-Alkylthio, $C_3$—$C_6$-Dialkoxyalkyl, $C_1$—$C_4$-Halogenalkylthio, $C_2$—$C_4$-Alkoxyalkyl, $C_3$—$C_6$-Cycloalkyl oder —$NR^{12}R^{13}$,

G eine Gruppe

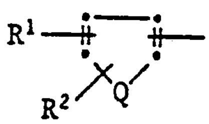 , oder 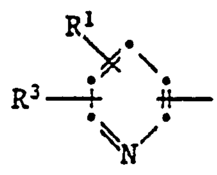 ,

$R^1$ Wasserstoff, Halogen, Nitro, $C_1$—$C_4$-Alkyl, $C_1$—$C_4$-Alkoxy, Trifluormethyl, Trifluormethoxy, Difluormethoxy, $C_1$—$C_4$-Alkylcarbonyl oder —$COOR^{14}$,

$R^2$ eine Gruppe

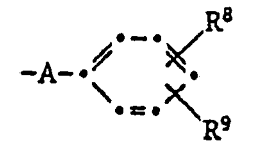 ,

$R^3$ eine Gruppe

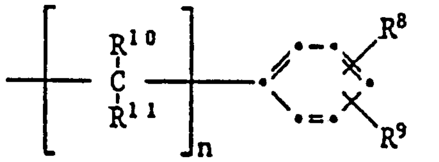 ,

Q Schwefel,

A Sauerstoff, Schwefel, —SO—, —$SO_2$—, oder —$C(R^{10}R^{11})_m$— m und n die Zahlen null, eins oder zwei,

$R^8$ Wasserstoff, $C_1$—$C_4$-Alkyl, $C_1$—$C_4$-Alkoxy, Halogen, $C_1$—$C_4$-Halogenalkyl, Nitro, —$COOR^{14}$-, $C_1$—$C_4$-Halogenalkoxy, —O—$CR^{15}R^{16}$—$COOR^{14}$ oder —O—$CR^{15}R^{16}$—CN,

$R^9$ Wasserstoff, Halogen, $C_1$—$C_4$-Alkyl, $C_1$—$C_4$-Alkoxy, $C_2$—$C_4$-Alkoxyalkyl oder $C_2$—$C_4$-Alkoxyalkoxy,

$R^{10}$ und $R^{11}$ unabhängig voneinander Wasserstoff oder $C_1$—$C_4$-Alkyl,

$R^{12}$ und $R^{13}$ unabhängig voneinander Wasserstoff oder $C_1$—$C_4$-Alkyl,

$R^{14}$ Wasserstoff, $C_1$—$C_4$-Alkyl, $C_2$—$C_4$-Alkenyl, $C_3$—$C_4$-Alkinyl oder durch $C_1$—$C_4$-Alkoxy, Halogen oder Phenyl substituiertes $C_1$—$C_4$-Alkyl und

$R^{15}$ und $R^{16}$ unabhängig voneinander Wasserstoff oder $C_1$—$C_4$-Alkyl bedeuten, sowie die Salze dieser Verbindungen.

2. Verbindungen gemäss Anspruch 1, dadurch gekennzeichnet, dass Z für Sauerstoff steht.

3. Verbindungen gemäss Anspruch 1, dadurch gekennzeichnet, dass $R^1$ für Wasserstoff, Cl, F, $CH_3$, $C_2H_5$, $CH_3O$, $CF_3$ $CH_3CO$ steht.

4. Verbindungen gemäss Anspruch 1, dadurch gekennzeichnet, dass $R^5$ und $R^6$ zusammen höchstens 4 Kohlenstoffatome enthalten und $R^4$ für Wasserstoff steht.

5. Verbindungen gemäss Anspruch 1, dadurch gekennzeichnet, dass G für 3-Phenylpyridin-2-yl steht.

6. Verbindungen gemäss Anspruch 1, dadurch gekennzeichnet, dass G für gegebenenfalls durch Cl, $CH_3$ oder $CH_3O$ substituiertes 3-Phenylthiophen-2-yl, 3-Phenoxythiophen-2-yl oder 2-Phenylthiophen-3-yl steht.

7. Verbindungen gemäss Anspruch 1, dadurch gekennzeichnet, dass Z Sauerstoff, $R^4$ Wasserstoff und G 3-Phenylpyridin-2-yl bedeuten und $R^5$ und $R^6$ zusammen höchstens 4 Kohlenstoffatome enthalten.

8. Verbindungen gemäss Anspruch 1, dadurch gekennzeichnet, dass G für 2-$C_1$—$C_4$-Alkoxycarbonyl-4-phenylthiophen-3-yl oder 2-$C_1$—$C_4$-Alkoxycarbonyl-5-phenylthiophen-3-yl steht.

9. Verbindungen gemäss Anspruch 1, dadurch gekennzeichnet, dass Z Sauerstoff, $R^4$ Wasserstoff und G 3-Phenylthiophen-2-yl, 2-Phenylthiophen-3-yl, oder 3-Phenoxythiophen-2-yl bedeuten und $R^5$ und $R^6$ zusammen höchstens 4 Kohlenstoffatome enthalten.

10. Verbindungen gemäss Anspruch 1, dadurch gekennzeichnet, dass Z Sauerstoff, $R^4$ Wasserstoff und G 2-$C_1$—$C_4$-Alkoxycarbonyl-4-phenylthiophen-3-yl oder 2-$C_1$—$C_4$-Alkoxycarbonyl-5-phenylthiophen-3-yl bedeuten und $R^5$ und $R^6$ zusammen höchstens 4-Kohlenstoffatome enthalten.

11. N-(2-Methoxycarbonyl-4-phenylthiophen-3-ylsulfonyl)-N'-(4-methoxy-6-methylpyrimidin-2-yl)-harnstoff gemäss Anspruch 1.

12. Verfahren zur Herstellung der Verbindungen der Formel I nach Anspruch 1, dadurch gekennzeichnet, dass man ein Sulfonamid der Formel II

$$G—SO_2—NH_2 \qquad (II),$$

worin G die unter Formel I gegebene Bedeutung hat, in Gegenwart einer Base mit einem Carbamat oder Thiocarbamat der Formel III

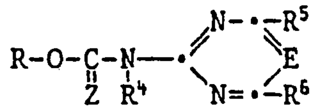

(III)

worin E, $R^4$, $R^5$, $R^6$ und Z die unter Formel I gegebene Bedeutung haben und R für Phenyl, Alkyl oder substituiertes Phenyl steht, umsetz und gegebenenfalls in ein Salz überführt.

13. Verfahren zur Herstellung der Verbindungen der Formel I nach Anspruch 1, dadurch gekennzeichnet, dass man ein Sulfonylcarbamat oder Sulfonylthiocarbamat der Formel IV

$$G—SO_2—NH—\underset{\underset{Z}{\|}}{C}—O—R \qquad (IV),$$

worin G und Z die unter Formel I gegebene Bedeutung haben und für Phenyl, Alkyl oder substituiertes Phenyl steht, mit einem Amin der Formel V

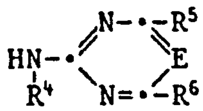

(V)

worin E, $R^4$, $R^5$ und $R^6$ die unter Formel I gegebene Bedeutung haben, umsetzt und gegebenenfalls in ein Salz überführt.

14. Verfahren zur Herstellung der Verbindungen der Formel I, nach Anspruch 1, dadurch gekennzeichnet, dass man ein Sulfonylisocyanat oder Sulfonylisothiocyanat der Formel VI

$$G—SO_2—N=C=Z \qquad (VI),$$

worin G und Z die unter Formel I gegebene Bedeutungen haben, mit einem Aminopyrmidin oder -triazin der im Anspruch 14 angegebenen Formel V umsetzt und gegebenenfalls in ein Salz überführt.

15. Verfahren zur Herstellung von Additionssalzen der Formel I gemäss einem der Ansprüche 12 bis 14, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

16. Ein herbizides und den Pflanzenwuchs hemmendes Mittel, dadurch gekennzeichnet, dass es neben Träger- und/oder anderen Zuschlagstoffen als Wirkstoff mindestens einen substituierten Sulfonylharnstoff der Formel I, Anspruch 1, enthält.

17. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

18. Verfahren zur Hemmung des Pflanzenwachstums, dadurch gekennzeichnet, dass man einen Wirkstoff der Formel I, gemäss Anspruch 1, oder ein Diesen Wirkstoff enthaltendes Mittel in einer Wirksamen Menge auf die Pflanzen oder deren Lebensraum appliziert.

19. Verfahren zur Beeinflussung des Pflanzenwachstums zum Zweck der Ertragssteigerung, dadurch gekennzeichnet, dass man einen Wirkstoff der formel I, gemäss Anspruch 1, oder ein diesen Wirkstoff enthaltendes Mittel in einer wirksamen Menge auf die Pflanzen oder deren Lebensraum applizierte.

20. Verfahren gemäss Anspruch 17, zur selektiven pre- oder post-emergenten Bekämpfung von Unkräutern in Nutzpflanzenkulturen.

21. Verfahren gemäss Anspruch 18, zur Unterdrückung des Pflanzenwachstums über das 2-Blattstadium hinaus, dadurch gekennzeichnet, dass die Wirkstoffe preemergent angewendet werden.

22. Verfahren gemäss Anspruch 20, in Kulturen von Reis.

**Patentansprüche für den Vertragsstaat: AT**

1. Ein herbzides und den Pflanzenwuchs hemmendes Mittel, dadurch gekennzeichnet, dass es neben Träger- und/oder anderen Zuschlagstoffen als Wirkstoff mindestens einen substituierten Sulfonylharnstoff der Formel I

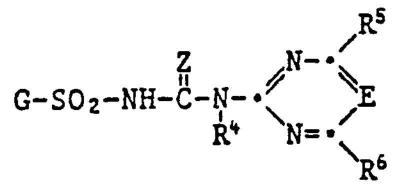

(I)

oder ein Salz davon enthält,
worin
E Stickstoff oder die Methinbrücke,
Z Sauerstoff oder Schwefel,
$R^4$ Wasserstoff oder $C_1$—$C_4$-Alkyl,
$R^5$ und $R^6$ unabhängig voneinander Wasserstoff, Halogen, $C_1$—$C_4$-Alkyl, $C_1$—$C_4$-Halogenalkyl, $C_1$—$C_4$-Alkoxy, $C_1$—$C_4$-Halogenalkoxy, $C_1$—$C_4$-Alkylthio, $C_3$—$C_6$-Dialkoxyalkyl, $C_1$—$C_4$-Halogenalkylthio, $C_2$—$C_4$-Alkoxyalkyl, $C_3$—$C_6$-Cycloalkyl oder —$NR^{12}R^{13}$,
G eine Gruppe

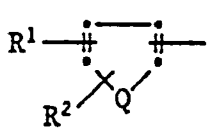

, oder

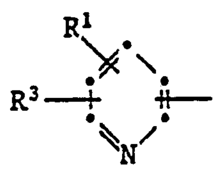

,

$R^1$ Wasserstoff, Halogen, Nitro, $C_1$—$C_4$-Alkyl, $C_1$—$C_4$-Alkoxy, Trifluormethyl, Trifluormethoxy, Difluormethoxy, $C_1$—$C_4$-Alkylcarbonyl oder —$COOR^{14}$,
$R^2$ eine Gruppe

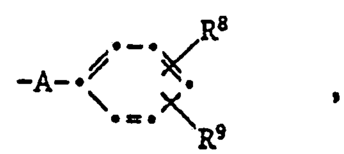

,

$R^3$ eine Gruppe

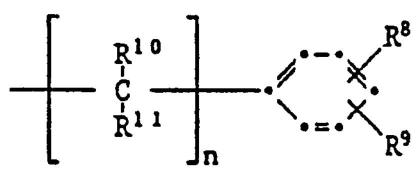

,

Q Schwefel,
A Sauerstoff, Schwefel, —SO—, —$SO_2$— oder —$C(R^{10}R^{11})_m$— m und n die Zahlen null, eins oder zwei,
$R^8$ Wasserstoff, $C_1$—$C_4$-Alkyl, $C_1$—$C_4$-Alkoxy, Halogen, $C_1$—$C_4$-Halogenalkyl, Nitro, —$COOR^{14}$, $C_1$—$C_4$-Halogenalkoxy, —O—$CR^{15}R^{16}$—$COOR^{14}$ oder —O—$CR^{15}R^{16}$—CN,
$R^9$ Wasserstoff, Halogen, $C_1$—$C_4$-Alkyl, $C_1$—$C_4$-Alkoxy, $C_2$—$C_4$-Alkoxyalkyl oder $C_2$—$C_4$-Alkoxyalkoxy,
$R^{10}$ und $R^{11}$ unabhängig voneinander Wasserstoff oder $C_1$—$C_4$-Alkyl,
$R^{12}$ und $R^{13}$ unabhängig voneinander Wasserstoff oder $C_1$—$C_4$-Alkyl,
$R^{14}$ Wasserstoff, $C_1$—$C_4$-Alkyl, $C_2$—$C_4$-Alkenyl, $C_3$—$C_4$-Alkinyl oder durch $C_1$—$C_4$-Alkoxy, Halogen oder Phenyl substituiertes $C_1$—$C_4$-Alkyl und
$R^{15}$ und $R^{16}$ unabhängig voneinander Wasserstoff oder $C_1$—$C_4$-Alkyl bedeuten.

2. Mittel gemäss Anspruch 1, dadurch gekennzeichnet, dass Z für Sauerstoff steht.

3. Mittel gemäss Anspruch 1, dadurch gekennzeichnet, dass $R^1$ für Wasserstoff, Cl, F, $CH_3$, $C_2H_5$, $CH_3O$, $CF_3$ $CH_3CO$ steht.

4. Mittel gemäss Anspruch 1, dadurch gekennzeichnet, dass $R^5$ und $R^6$ zusammen höchstens 4 Kohlenstoffatome enthalten und $R^4$ für Wasserstoff steht.

5. Mittel gemäss Anspruch 1, dadurch gekennzeichnet, dass G für 3-Phenylpyridin-2-yl steht.

6. Mittel gemäss Anspruch 1, dadurch gekennzeichnet, dass G für gegebenenfalls durch Cl, $CH_3$ oder $CH_3O$ substituiertes 3-Phenylthiophen-2-yl, 3-Phenoxythiophen-2-yl oder 2-Phenylthiophen-3-yl steht.

7. Mittel gemäss Anspruch 1, dadurch gekennzeichnet, dass Z Sauerstoff, $R^4$ Wasserstoff und G 3-Phenylpyridin-2-yl bedeuten und $R^5$ und $R^6$ zusammen höchstens 4 Kohlenstoffatome enthalten.

8. Mittel gemäss Anspruch 1, dadurch gekennzeichnet, dass G für 2-$C_1$—$C_4$-Alkoxycarbonyl-4-phenylthiophen-3-yl oder 2-$C_1$—$C_4$-Alkoxycarbonyl-5-phenylthiophen-3-yl steht.

9. Mittel gemäss Anspruch 1, dadurch gekennzeichnet, dass Z Sauerstoff, $R^4$ Wasserstoff und G 3-Phenylthiophen-2-yl, 2-Phenylthiophen-3-yl, oder 3-Phenoxythiophen-2-yl bedeuten und $R^5$ und $R^6$ zusammen höchstens 4 Kohlenstoffatome enthalten.

10. Mittel gemäss Anspruch 1, dadurch gekennzeichnet, dass Z Sauerstoff, $R^4$ Wasserstoff und G 2-$C_1$—$C_4$-Alkoxycarbonyl-4-phenylthiophen-3-yl oder 2-$C_1$—$C_4$-Alkoxycarbonyl-5-phenylthiophen-3-yl bedeuten und $R^5$ und $R^6$ zusammen höchstens 4-Kohlenstoffatome enthalten.

11. Mittel gemäss Anspruch 1, dadurch gekennzeichnet, dass es als Wirkstoff N-(2-Methoxycarbonyl-4-phenylthiophen-3-ylsulfonyl)-N'-(4-methoxy-6-methylpyrimidin-2-yl)-harnstoff enthält.

12. Verfahren zur Herstellung der Verbindungen der Formel I nach Anspruch 1, dadurch gekennzeichnet, dass man ein Sulfonamid der Formel II

$$G-SO_2-NH_2 \qquad (II),$$

worin G die unter Formel I gegebene Bedeutung hat, in Gegenwart einer Base mit einem Carbamat oder Thiocarbamat der Formel III

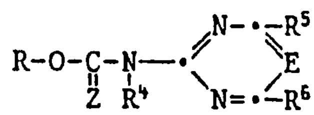

(III)

worin E, $R^4$, $R^5$, $R^6$ und Z die unter Formel I gegebene Bedeutung haben und R für Phenyl, Alkyl oder substituiertes Phenyl steht, umsetz und gegebenenfalls in ein Salz überführt.

13. Verfahren zur Herstellung der Verbindungen der Formel I nach Anspruch 1, dadurch gekennzeichnet, dass man ein Sulfonylcarbamat oder Sulfonylthiocarbamat der Formel IV

$$G-SO_2-NH-\underset{\underset{Z}{\parallel}}{C}-O-R \qquad (IV),$$

worin G und Z die unter Formel I gegebene Bedeutung haben und für Phenyl, Alkyl oder substituiertes Phenyl steht, mit einem Amin der Formel V

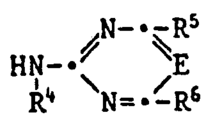

(V)

worin E, $R^4$, $R^5$ und $R^6$ die unter Formel I gegebene Bedeutung haben, umsetzt und gegebenenfalls in ein Salz überführt.

14. Verfahren zur Herstellung der Verbindungen der Formel I, nach Anspruch 1, dadurch gekennzeichnet, dass man ein Sulfonylisocyanat oder Sulfonylisothiocyanat der Formel VI

$$G-SO_2-N=C=Z \qquad (VI),$$

worin G und Z die unter Formel I gegebene Bedeutung haben, mit einem Aminopyrmidin oder -triazin der im Anspruch 13 angegebenen Formel V umsetzt und gegebenenfalls in ein Salz überführt.

15. Verfahren zur Herstellung von Additionssalzen der Formel I gemäss einem der Ansprüche 12 bis 14, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

16. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

17. Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

18. Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

19. Verfahren gemäss Anspruch 16, zur selektiven pre- oder postemergenten Bekämpfung von Unkräutern in Nutzpflanzenkulturen.

20. Verfahren gemäss Anspruch 17, zur Unterdrückung des Pflanzenwachstums über das 2-Blattstadium hinaus, dadurch gekennzeichnet, dass die Wirkstoffe preemergent angewendet werden.

21. Verfahren gemäss Anspruch 19 in Kulturen von Reis.

**Revendications pour les Etats contractants: BE CH DE FR GB IT LI NL SE**

1. Sulfonylurées de formule I

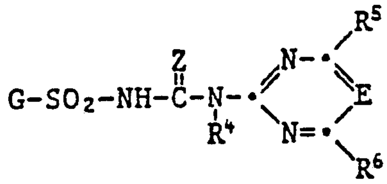

(I)

où

E représente l'azote ou le pont méthine,

Z représente l'oxygène ou le soufre,

$R^4$ représente l'hydrogène ou un alkyle en $C_1$—$C_4$,

$R^5$ et $R^6$, indépendamment l'un de l'autre, représentent l'hydrogène, un halogène, un alkyle en $C_1$—$C_4$, un halogénoalkyle, en $C_1$—$C_4$, un alcoxy en $C_1$—$C_4$, un halogénoalcoxy en $C_1$—$C_4$, un alkylthio en $C_1$—$C_4$, un dialcoxyalkyle en $C_3$—$C_6$, un halogénoalkylthio en $C_1$—$C_4$, un alcoxyalkyle en $C_2$—$C_4$, un cycloalkyle en $C_3$—$C_6$ ou —$NR^{12}R^{13}$,

G représente un groupe

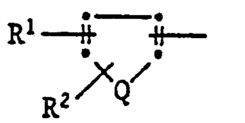

, ou

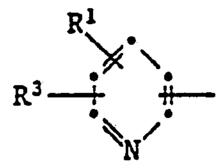

,

$R^1$ représente l'hydrogène, un halogène, un nitro, un alkyle en $C_1$—$C_4$, un alcoxy en $C_1$—$C_4$, le trifluorométhyle, le trifluorométhoxy, le difluorométhoxy, un alkyl$C_1$—$C_4$carbonyle ou —$COOR^{14}$,

$R^2$ représente un groupe

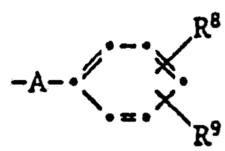

,

$R^3$ représente un groupe

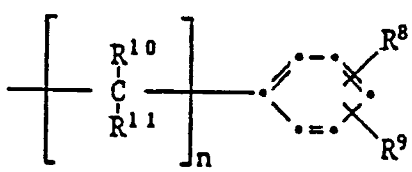

,

Q représente le soufre,

A représente l'oxygène, le soufre, —SO—, —$SO_2$— ou —$(CR^{10}R^{11})_m$—

m et n représentent les chiffres 0, un ou deux,

$R^8$ représente l'hydrogène, un alkyle en $C_1$—$C_4$, un alcoxy en $C_1$—$C_4$, un halogène, un halogénoalkyle en $C_1$—$C_4$, un nitro, —$COOR^{14}$, un halogénoalcoxy en $C_1$—$C_4$, —O—$CR^{15}R^{16}$—$COOR^{14}$ ou —O—$CR^{15}R^{16}$—CN,

$R^9$ représente l'hydrogène, un halogène, un alkyle en $C_1$—$C_4$, un alcoxy en $C_1$—$C_4$, un alcoxyalkyle en $C_2$—$C_4$ ou un alcoxyalcoxy en $C_2$—$C_4$,

$R^{10}$ et $R^{11}$, indépendamment l'un de l'autre, représentent l'hydrogène ou un alkyle en $C_1$—$C_4$,

$R^{12}$ et $R^{13}$, indépendamment l'un de l'autre, représentent l'hydrogène ou un alkyle en $C_1$—$C_4$,

$R^{14}$ représente l'hydrogène, un alkyle en $C_1$—$C_4$, un alkényle en $C_2$—$C_4$, un alkinyle en $C_3$—$C_4$ ou un alkyle en $C_1$—$C_4$ substitué par un alcoxy en $C_1$—$C_4$ un halogène ou un phényle et

$R^{15}$ et $R^{16}$, indépendamment l'un de l'autre, représentent l'hydrogène ou un alkyle en $C_1$—$C_4$, ainsi que les sels de ces composés.

2. Composés selon la revendication 1, caractérisés en ce que Z représente l'oxygène.

3. Composés selon la revendication 1, caractérisés en ce que $R^1$ représente l'hydrogène, Cl, F, $CH_3$, $C_2H_5$, $CH_3O$, $CF_3$, $CH_3CO$.

4. Composés selon la revendication 1, caractérisés en ce que $R^5$ et $R^6$ ensemble comportent au plus 4 atomes de carbone, et en ce que $R^4$ représente l'hydrogène.

5. Composés selon la revendication 1, caractérisés en ce que G représente le 3-phénylpyridine-2-yle.

6. Composés selon la revendication 1, caractérisés en ce que G représente le 3-phénylthiophène-2-yle, le 3-phénoxythiophène-2-yle, ou le 2-phénylthiophène-3-yle, éventuellement substitués par Cl, $CH_3$ ou $CH_3O$.

7. Composés selon la revendication 1, caractérisés en ce que Z représente l'oxygène, $R^4$ représente l'hydrogène et G représente le 3-phénylpyridine-2-yle, et $R^5$ et $R^6$ ensemble comportent au plus 4 atomes de carbone.

8. Composés selon la revendication 1, caractérisés en ce que G représente un 2-alcoxy$C_1$—$C_4$carbonyl-4-phénylthiophène-3-yle ou un 2-alcoxy$C_1$—$C_4$carbonyl-5-phénylthiophène-3-yle.

9. Composés selon la revendication 1, caractérisés en ce que Z représente l'oxygène, $R^4$ représente l'hydrogène et G représente le 3-phénylthiophène-2-yle, le 2-phénylthiophène-3-yle ou le 3-phénoxythiophène-2-yle, et $R^5$ et $R^6$ ensemble comportent au plus 4 atomes de carbone.

10. Composés selon la revendication 1, caractérisés en ce que Z représente l'oxygène, $R^4$ représente l'hydrogène et G représente un 2-alcoxy$C_1$—$C_4$carbonyl-4-phénylthiophène-3-yle ou un 2-alcoxy$C_1$—$C_4$-carbonyl-5-phénylthiophène-3-yle, et $R^5$ et $R^6$ ensemble comportent au plus 4 atomes de carbone.

11. N-(2-méthoxycarbonyl-4-phénylthiophène-3-ylsulfonyl)-N'-(4-méthoxy-6-méthylpyrimidine-2-yl)urée selon la revendication 1.

12. Procédé pour préparer les composés de formule I selon la revendication 1, caractérisé en ce que l'on fait réagir, en présence d'une base, un sulfonamide de formule II

$$G—SO_2—NH_2 \qquad (II),$$

où G a la signification donnée sous la formule I, avec un carbamate ou un thiocarbamate de formule III

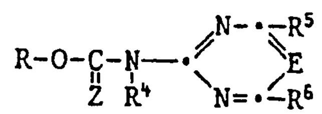

(III)

où E, $R^4$, $R^5$, $R^6$ et Z ont la signification donnée sous la formule I, et R représente le phényle, un alkyle ou un phényle substitué, et en ce qu'on transforme éventuellement en un sel.

13. Procédé pour préparer les composés de formule I selon la revendication 1, caractérisé en ce que l'on fait réagir un sulfonylcarbamate ou un sulfonylthiocarbamate de formule IV

$$G—SO_2—NH—\underset{\underset{Z}{\|}}{C}—O—R \qquad (IV),$$

où G et Z ont la signification donnée sous la formule I et R représente le phényle, un alkyle ou un phényle substitué, avec une amine de formule V

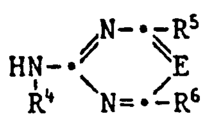

(V)

où E, $R^4$, $R^5$ et $R^6$ ont la signification donnée sous la formule I, et en ce qu'on transforme éventuellement en un sel.

14. Procédé pour préparer les composés de formule I, selon la revendication 1, caractérisé en ce que l'on fait réagir un sulfonylisocyanate ou un sulfonylisothiocyanat de formule VI

$$G—SO_2—N=C=Z \qquad (VI),$$

où G et Z ont la signification donnée sous la formule I, avec une aminopyrimidine ou aminotriazine de formule V donnée à la revendication 13, et en ce qu'on transforme éventuellement en un sel.

15. Procédé pour préparer des sels d'addition de formule I selon l'une des revendications 12 à 14, caractérisé en ce que l'on fait réagir une sulfonylurée de formule I avec une amine, un hydroxyde de métal alcalin ou alcalino-terreux, ou une base d'ammonium quaternaire.

16. Moyen herbicide et limitant la croissance des plantes, caractérisé en ce qu'il comporte, en plus de matières supports et/ou d'autres adjuvants, au moins une sulfonylurée substituée de formule I, revendication 1, comme matière active.

17. Procédé pour lutter contre la croissance non désirée de plantes, caractérisé en ce qu'on applique,

en quantité efficace, sur les plantes ou leurs biotopes, une matière active de formule I, selon la revendication 1, ou un moyen comportant cette matière active.

18. Procédé pour limiter la croisance des plantes, caractérisé en ce que l'on applique une matière active de formule I, selon la revendication 1, ou un moyen contenant cette matière active, dans une quantité efficace, sur les plantes ou leurs biotopes.

19. Procédé pour influencer la croissance des plants dans le but d'augmenter le rendement, caractérisé en ce que l'on applique une matière active de fomule I, selon la revendication 1, ou un moyen contenant cette matière active, dans une quantité efficace, sur les plantes ou leurs biotopes.

20. Procédé selon la revendication 17, pour lutter de façon sélective contre les mauvaises herbes, avant ou après leurs apparition, dans les cultures de plantes utiles.

21. Procédé selon la revendication 18, pour arrêter la croissance des plantes, jusqu'au stade de deux feuilles, caractérisé en ce qu'on utilise la matière active avant l'apparition des plantes.

22. Procédé selon la revendication 20 dans les cultures de riz.

**Revendications pour l'Etat contractant: AT**

1. Moyen herbicide et limitant la croissance des plantes caractérisé en ce que, en plus de matières supports et/ou d'autres adjuvants, il comporte comme matière active au moins une sulfonylurée substituée de formule I,

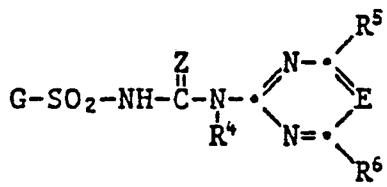

(I)

ou un sel de celle-ci,
où

E représente l'azote ou le pont méthine,
Z représente l'oxygène ou le soufre,
$R^4$ représent l'hydrogène ou un alkyle en $C_1$—$C_4$,
$R^5$ et $R^6$, indépendamment l'un de l'autre, représentent l'hydrogène, un halogène, un alkyle en $C_1$—$C_4$, un halogénoalkyle, en $C_1$—$C_4$, un alcoxy en $C_1$—$C_4$, un halogénoalcoxy en $C_1$—$C_4$, un alkylthio en $C_1$—$C_4$, un dialcoxyalkyle en $C_3$—$C_6$, un halogénoalkylthio en $C_1$—$C_4$, un alcoxyalkyle en $C_2$—$C_4$, un cycloalkyle en $C_3$—$C_6$ ou —$NR^{12}R^{13}$,
G représente un groupe

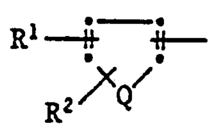 , ou 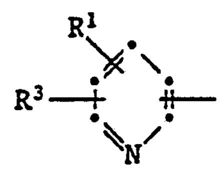 ,

$R^1$ représente l'hydrogène, un halogène, un nitro, un alkyle en $C_1$—$C_4$, un alcoxy en $C_1$—$C_4$, le trifluorométhyle, le trifluorométhoxy, le difluorométhoxy, un alkyl$C_1$—$C_4$carbonyle ou —$COOR^{14}$,
$R^2$ représente un groupe

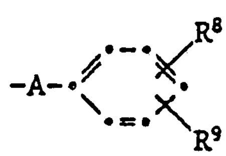 ,

$R^3$ représente un groupe

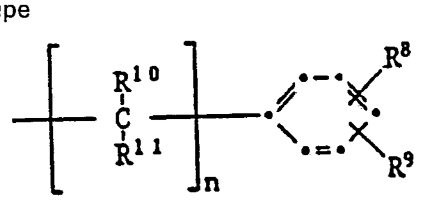 ,

Q représente le soufre,
A représente l'oxygène, le soufre, —SO—, —$SO_2$— ou —$(CR^{10}R^{11})_m$—
m et n représentent les chiffres 0, un ou deux,

$R^8$ représente l'hydrogène, un alkyle en $C_1$—$C_4$, un alcoxy en $C_1$—$C_4$, un halogène, un halogénoalkyle en $C_1$—$C_4$, un nitro, —$COOR^{14}$, un halogénoalcoxy en $C_1$—$C_4$, —O—$CR^{15}R^{16}$—$COOR^{14}$ ou —O—$CR^{15}R^{16}$—CN,

$R^9$ représente l'hydrogène, un halogène, un alkyle en $C_1$—$C_4$, un alcoxy en $C_1$—$C_4$, un alcoxyalkyle en $C_2$—$C_4$ ou un alcoxyalkoxy en $C_2$—$C_4$,

$R^{10}$ et $R^{11}$, indépendamment l'un de l'autre, représentent l'hydrogène ou un alkyle en $C_1$—$C_4$,

$R^{12}$ et $R^{13}$, indépendamment l'un de l'autre, représentent l'hydrogène ou un alkyle en $C_1$—$C_4$,

$R^{14}$ représente l'hydrogène, un alkyle en $C_1$—$C_4$, un alkényle en $C_2$—$C_4$, un alkinyle en $C_3$—$C_4$ ou un alkyle en $C_1$—$C_4$ substitué par un alcoxy en $C_1$—$C_4$ un halogène ou un phényle et

$R^{15}$ et $R^{16}$, indépendamment l'un de l'autre, représentent l'hydrogène ou un alkyle en $C_1$—$C_4$.

2. Moyen selon la revendication 1, caractérisé en ce que Z représente l'oxygène.

3. Moyen selon la revendication 1, caractérisé en ce que $R^1$ représente l'hydrogène, Cl, F, $CH_3$, $C_2H_5$, $CH_3O$, $CF_3$, $CH_3CO$.

4. Moyen selon la revendication 1, caractérisé en ce que $R^5$ et $R^6$ ensemble comportent au plus quatre atomes de carbone et en ce que $R^4$ représente l'hydrogène.

5. Moyen selon la revendication 1, caractérisé en ce que G représente le 3-phénylpyridine-2-yl.

6. Moyen selon la revendication 1, caractérisé en ce que G représente le 3-phénylthiophène-2-yle, le 3-phénoxythiophène-2-yle ou le 2-phénylthiophène-3-yle, éventuellement substitués par Cl, $CH_3$, ou $CH_3O$.

7. Moyen selon la revendication 1, caractérisé en ce que Z représente l'oxygène, $R^4$ représente l'hydrogène et G représente le 3-phénylpyridine-2-yle, et en ce que $R^5$ et $R^6$ ensemble comportent au plus 4 atomes de carbone.

8. Moyen selon la revendication 1, caractérisé en ce que G représente un 2-alcoxy$C_1$—$C_4$carbonyl-4-phénylthiophène-3-yle ou un 2-alcoxy$C_1$—$C_4$carbonyl-5-phénylthiophène-3-yle.

9. Moyen selon la revendication 1, caractérisé en ce que Z représente l'oxygène, $R^4$ représente l'hydrogène et G représente le 3-phénylthiophène-2-yle, le 2-phénylthiophène-3-yle ou le 3-phénoxythiophène-2-yle, et en ce que $R^5$ et $R^6$ ensemble comportent au plus 4 atomes de carbone.

10. Moyen selon la revendication 1, caractérisé en ce que Z représente l'oxygène, $R^4$ représente l'hydrogène et G représente un 2-alcoxy$C_1$—$C_4$carbonyl-4-phénylthiophène-3-yle ou un 2-alcoxy$C_1$—$C_4$-carbonyl-5-phénylthiophène-3-yle, et $R^5$ et $R^6$ ensemble comportent au plus 4 atomes de carbone.

11. Moyen selon la revendication 1, caractérisé en ce qu'il comporte comme matière active la N-(2-méthoxycarbonyl-4-phénylthiophène-3-ylsulfonyl)-N'-(4-méthoxy-6-méthylpyrimidine-2-yl)urée.

12. Procédé pour préparer les composés de formule I selon la revendication 1, caractérisé en ce que l'on fait réagir un sulfonamide, de formule II

$$G—SO_2—NH_2 \qquad (II),$$

où G a la signification donnée à la formule I, en présence d'une base, avec un carbamate ou un thiocarbamate de formule III

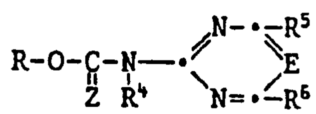

(III)

où E, $R^4$, $R^5$, $R^6$ et Z ont la signification donnée sous la formule I et R représente le phényle, un alkyle ou un phényle substitué, et en ce qu'on transforme éventuellement en un sel.

13. Procédé pour préparer les composés de formule I selon la revendication 1, caractérisé en ce que l'on fait réagir un sulfonylcarbamate ou un sulfonylthiocarbamate de formule IV

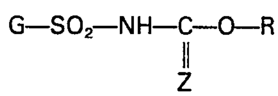

(IV),

où G et Z ont la signification donnée sous la formule I et R représentent le phényle, un alkyle ou un phényle substitué, avec une amine de formule V

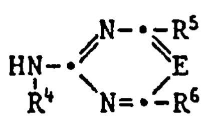

(V)

où E, $R^4$, $R^5$ et $R^6$ ont la signification donnée sous la formule I, et en ce qu'on transforme éventuellement en un sel.

14. Procédé pour préparer les composés de formule I, selon la revendication 1, caractérisé en ce que l'on fait réagir un sulfonylisocyanate ou un sulfonylisothiocyanate de formule VI

$$G—SO_2—N=C=Z \qquad (VI),$$

où G et Z ont la signification donnée sous la formule I, avec une aminopyrimidine ou une aminotriazine de formule V correspondant à la revendication 13, et en ce qu'on transforme éventuellement en un sel.

15. Procédé pour préparer les sels d'addition de formule I selon l'une des revendications 12 à 14, caractérisé en ce que l'on fait réagir une sulfonylurée de formule I avec une amine, un hydroxyde de métal alcalin ou alcalino-terreux, ou une base d'ammonium quaternaire.

16. Procédé pour lutter contre la croissance non désirée de plantes, caractérisé en ce que l'on applique une matière active de formule I, selon la revendication 1, ou un moyen contenant cette matière active, dans une quantité efficace, sur les plantes ou sur leur biotopes.

17. Procédé pour ralentir la croissance des plantes, caractérisé en ce que l'on applique une matière active de formule I, selon la revendication 1, ou un moyen contenant cette matière active, dans une quantité efficace, sur les plantes ou sur leur biotopes.

18. Procédé pour influencer la croissance des plantes dans le but d'augmenter le rendement, caractérisé en ce que l'on applique une matière active de formule I, selon la revendication 1, ou un moyen contenant cette matière active, dans une quantité efficace, sur les plantes ou sur leur biotopes.

19. Procédé selon la revendication 16, pour lutter de façon sélective contre les mauvaises herbes, avant ou après leur apparition, dans les cultures de plantes utiles.

20. Procédé selon la revendication 17, pour retenir la croissance des plantes jusqu'au stade de deux feuilles, caractérisé en ce que l'on utilise les matières actives avant l'apparition des plantes.

21. Procédé selon la revendication 19, dans les cultures du riz.

**Claims for the Contracting States: BE CH DE FR GB IT LI NL SE**

1. Sulfonylureas of formula I

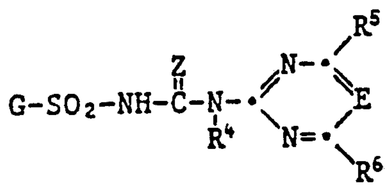

(I)

wherein

E is nitrogen or the methine bridge,

Z is oxygen or sulfur,

$R^4$ is hydrogen or $C_1$—$C_4$alkyl,

$R^5$ and $R^6$ are each independently hydrogen, halogen, $C_1$—$C_4$alkyl, $C_1$—$C_4$haloalkyl, $C_1$—$C_4$alkoxy, $C_1$—$C_4$haloalkoxy, $C_1$—$C_4$alkylthio, $C_3$—$C_6$dialkoxyalkyl, $C_1$—$C_4$haloalkylthio, $C_2$—$C_4$alkoxyalkyl, $C_3$—$C_6$cycloalkyl or —$NR^{12}R^{13}$,

G is a

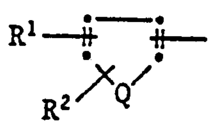

, or

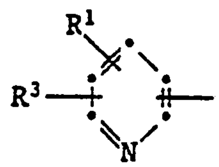

group,

$R^1$ is hydrogen, halogen, nitro, $C_1$—$C_4$alkyl, $C_1$—$C_4$alkoxy, trifluoromethyl, trifluoromethoxy, difluoromethoxy, $C_1$—$C_4$alkylcarbonyl or —$COOR^{14}$,

$R^2$ is a

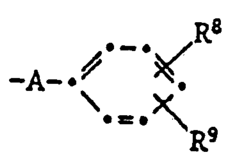

group,

$R^3$ is a

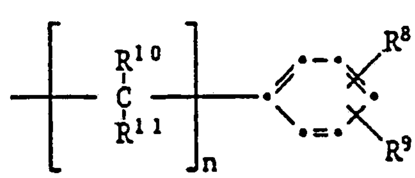

group,

Q is sulfur,

A is oxygen, sulfur, —SO—, —$SO_2$— or —$(CR^{10}R^{11})_m$—,

m and n are 0, 1 or 2,

$R^8$ is hydrogen, $C_1$—$C_4$alkyl, $C_1$—$C_4$alkoxy, halogen, $C_1$—$C_4$haloalkyl, nitro, —$COOR^{14}$, $C_1$—$C_4$haloalkoxy, —O—$CR^{15}R^{16}COOR^{14}$ or —O—$CR^{15}R^{16}$—CN,

$R^9$ is hydrogen, halogen, $C_1$—$C_4$alkyl, $C_1$—$C_4$alkoxy, $C_2$—$C_4$alkoxyalkyl or $C_2$—$C_4$alkoxyalkoxy,

$R^{10}$ and $R^{11}$ are each independently hydrogen or $C_1$—$C_4$-alkyl,

$R^{12}$ and $R^{13}$ are each independently hydrogen or $C_1$—$C_4$alkyl,

$R^{14}$ is hydrogen, $C_1$—$C_4$alkyl, $C_2$—$C_4$alkenyl, $C_3$—$C_4$alkynyl, or is $C_1$—$C_4$alkyl substituted by $C_1$—$C_4$alkoxy, halogen or by phenyl, and

$R^{15}$ and $R^{16}$ are each independently hydrogen or $C_1$—$C_4$alkyl, and salts of those compounds.

2. Compounds according to claim 1, wherein Z is oxygen.

3. Compounds according to claim 1, wherein $R^1$ is hydrogen, Cl, F, $CH_3$, $C_2H_5$, $CH_3O$, $CF_3$ or $CH_3CO$.

4. Compounds according to claim 1, wherein $R^5$ and $R^6$ together contain not more than 4 carbon atoms and $R^4$ is hydrogen.

5. Compounds according to claim 1, wherein G is 3-phenylpyridin-2-yl.

6. Compounds according to claim 1, wherein G is 3-phenylthiophen-2-yl, 3-phenoxythiophen-2-yl or 2-phenylthiophen-3-yl, each unsubstituted or substituted by Cl, $CH_3$ or $CH_3O$.

7. Compounds according to claim 1, wherein Z is oxygen, $R^4$ is hydrogen and G is 3-phenylpyridin-2-yl and $R^5$ and $R^6$ together contain not more than 4 carbon atoms.

8. Compounds according to claim 1, wherein G is 2-$C_1$—$C_4$alkoxycarbonyl-4-phenylthiophen-3-yl or 2-$C_1$—$C_4$alkoxycarbonyl-5-phenylthiophen-3-yl.

9. Compounds according to claim 1, wherein Z is oxygen, $R^4$ is hydrogen and G is 3-phenylthiophen-2-yl, 2-phenylthiophen-3-yl or 3-phenoxythiophen-2-yl and $R^5$ and $R^6$ together contain not more than 4 carbon atoms.

10. Compounds according to claim 1, wherein Z is oxygen, $R^4$ is hydrogen and G is 2-$C_1$—$C_4$alkoxycarbonyl-4-phenylthiophen-3-yl or 2-$C_1$—$C_4$alkoxycarbonyl-5-phenylthiophen-3-yl, and $R^5$ and $R^6$ together contain not more than 4 carbon atoms.

11. N-(2-Methoxycarbonyl-4-phenylthiophen-3-ylsulfonyl)-N'-(4-methoxy-6-methylpyrimidin-2-yl)urea according to claim 1.

12. A process for the preparation of compounds of formula I according to claim 1, which process comprises reacting a sulfonamide of formula II

$$G—SO_2—NH_2 \qquad (II)$$

wherein G is as defined for formula I, with a carbamate or thiocarbamate of formula III

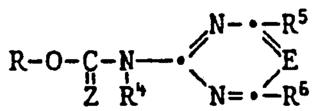

(III)

wherein E, $R^4$, $R^5$, $R^6$ and Z are as defined for formula I and R is phenyl, alkyl or substituted phenyl, in the presence of a base, and optionally converting the resulting compound of formula I into a salt.

13. A process for the preparation of compounds of formula I according to claim 1, which process comprises reacting a sulfonyl carbamate or sulfonyl thiocarbamate of formula IV

$$G—SO_2—NH—\underset{\underset{Z}{\|}}{C}—O—R \qquad (IV)$$

wherein G and Z are as defined for formula I and R is phenyl, alkyl or substituted phenyl, with an amine of formula V

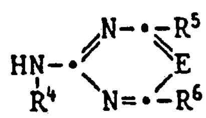

(V)

wherein E, $R^4$, $R^5$ and $R^6$ are as defined for formula I, and optionally converting the resulting compound of formula I into a salt.

14. A process for the preparation of compounds of formula I according to claim 1, which process comprises reacting a sulfonyl isocyanate or sulfonyl isothiocyanate of formula VI

$$G—SO_2—N{=}C{=}Z \qquad (VI)$$

wherein G and Z are as defined for formula I, with an aminopyrimidine or aminotriazine of formula V according to claim 13, and optionally converting the resulting compound of formula I into a salt.

15. A process for the preparation of addition salts of formula I according to any one of claims 12 to 14, which process comprises reacting a sulfonylurea of formula I with an amine, an alkali metal hydroxide or an alkaline earth metal hydroxide or with a quaternary ammonium base.

16. A herbicidal and plant growth-inhibiting composition which contains, as active ingredient, at least one substituted sulfonylurea of formula I according to claim 1, together with carriers and/or other adjuvants.

17. A method of controlling undesired plant growth, which method comprises applying to the plants or to the locus thereof an effective amount of a compound of formula I according to claim 1, or of a composition containing such a compound.

18. A method of inhibiting plant growth, which method comprises applying to the plants or to the locus thereof an effective amount of a compound of formula I according to claim 1, or of a composition containing such a compound.

19. A method of influencing plant growth in order to increase yield, which method comprises applying to the plants or to the locus thereof an effective amount of a compound of formula I according to claim 1, or of a composition containing such a compound.

20. A method according to claim 17, of selectively controlling weeds pre- or post-emergence in crops of useful plants.

21. A method according to claim 18 of inhibiting plant growth beyond the 2-leaf stage, which method comprises applying the active ingredients pre-emergence.

22. A method according to claim 20, wherein the crops are rice crops.

**Claims for the Contracting State: AT**

1. A herbicidal and plant growth-inhibiting composition, which contains as active ingredient at least one substituted sulfonylurea of formula I

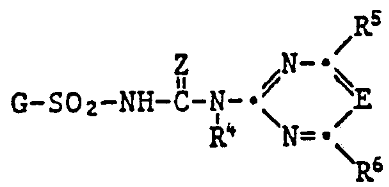

(I)

or a salt thereof, wherein

E is nitrogen or the methine bridge,

Z is oxygen or sulfur,

$R^4$ is hydrogen or $C_1$—$C_4$alkyl,

$R^5$ and $R^6$ are each independently hydrogen, halogen, $C_1$—$C_4$alkyl, $C_1$—$C_4$haloalkyl, $C_1$—$C_4$alkoxy, $C_1$—$C_4$haloalkoxy, $C_1$—$C_4$alkylthio, $C_3$—$C_6$dialkoxyalkyl, $C_1$—$C_4$haloalkylthio, $C_2$—$C_4$alkoxyalkyl, $C_3$—$C_6$cycloalkyl or —$NR^{12}R^{13}$,

G is a

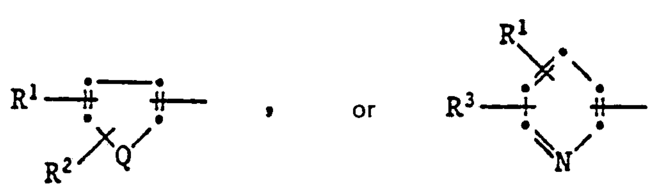

group,

$R^1$ is hydrogen, halogen, nitro, $C_1$—$C_4$alkyl, $C_1$—$C_4$alkoxy, trifluoromethyl, trifluoromethoxy, difluoromethoxy, $C_1$—$C_4$alkylcarbonyl or —$COOR^{14}$,

$R^2$ is a

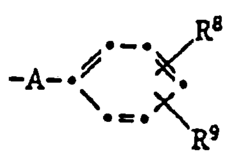

group,

$R^3$ is a

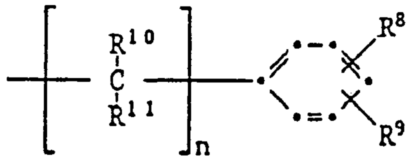

group,

Q is sulfur,

A is oxygen, sulfur, —SO—, $—SO_2—$ or $—(CR^{10}R^{11})_m—$,

m and n are 0, 1 or 2,

$R^8$ is hydrogen, $C_1—C_4$alkyl, $C_1—C_4$alkoxy, halogen, $C_1—C_4$haloalkyl, nitro, $—COOR^{14}$, $C_1—C_4$haloalkoxy, $—O—CR^{15}R^{16}COOR^{14}$ or $—O—CR^{15}R^{16}—CN$,

$R^9$ is hydrogen, halogen, $C_1—C_4$alkyl, $C_1—C_4$alkoxy, $C_2—C_4$alkoxyalkyl or $C_2—C_4$alkoxyalkoxy,

$R^{10}$ and $R^{11}$ are each independently hydrogen or $C_1—C_4$-alkyl,

$R^{12}$ and $R^{13}$ are each independently hydrogen or $C_1—C_4$alkyl,

$R^{14}$ is hydrogen, $C_1—C_4$alkyl, $C_2—C_4$alkenyl, $C_3—C_4$alkynyl, or is $C_1—C_4$alkyl substituted by $C_1—C_4$alkoxy, halogen or by phenyl, and

$R^{15}$ and $R^{16}$ are each independently hydrogen or $C_1—C_4$alkyl, together with carriers and/or other adjuvants.

2. A composition according to claim 1, wherein Z is oxygen.

3. A composition according to claim 1, wherein $R^1$ is hydrogen, Cl, F, $CH_3$, $C_2H_5$, $CH_3O$, $CF_3$ or $CH_3CO$.

4. A composition according to claim 1, wherein $R^5$ and $R^6$ together contain not more than 4 carbon atoms and $R^4$ is hydrogen.

5. A composition according to claim 1, wherein G is 3-phenylpyridin-2-yl.

6. A composition according to claim 1, wherein G is 3-phenylthiophen-2-yl, 3-phenoxythiophen-2-yl or 2-phenylthiophen-3-yl, each unsubstituted or substituted by Cl, $CH_3$ or $CH_3O$.

7. A composition according to claim 1, wherein Z is oxygen, $R^4$ is hydrogen and G is 3-phenylpyridin-2-yl and $R^5$ and $R^6$ together contain not more than 4 carbon atoms.

8. A composition according to claim 1, wherein G is 2-$C_1—C_4$alkoxycarbonyl-4-phenylthiophen-3-yl or 2-$C_1—C_4$alkoxycarbonyl-5-phenylthiophen-3-yl.

9. A composition according to claim 1, wherein Z is oxygen, $R^4$ is hydrogen and G is 3-phenylthiophen-2-yl, 2-phenylthiophen-3-yl or 3-phenoxythiophen-2-yl and $R^5$ and $R^6$ together contain not more than 4 carbon atoms.

10. A composition according to claim 1, wherein Z is oxygen, $R^4$ is hydrogen and G is 2-$C_1—C_4$alkoxycarbonyl-4-phenylthiophen-3-yl or 2-$C_1—C_4$alkoxycarbonyl-5-phenylthiophen-3-yl, and $R^5$ and $R^6$ together contain not more than 4 carbon atoms.

11. A composition according to claim 1, which contains as active ingredient N-(2-methoxycarbonyl-4-phenylthiophen-3-ylsulfonyl)-N'-(4-methoxy-6-methylpyrimidin-2-yl)urea.

12. A process for the preparation of compounds of formula I according to claim 1, which process comprises reacting a sulfonamide of formula II

$$G—SO_2—NH_2 \qquad (II)$$

wherein G is as defined for formula I, with a carbamate or thiocarbamate of formula III

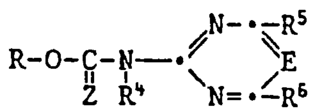

(III)

wherein E, $R^4$, $R^5$, $R^6$ and Z are as defined for formula I and R is phenyl, alkyl or substituted phenyl, in the presence of a base, and optionally converting the resulting compound of formula I into a salt.

13. A process for the preparation of compounds of formula I according to claim 1, which process comprises reacting a sulfonyl carbamate or sulfonyl thiocarbamate of formula IV

$$G—SO_2—NH—\underset{\underset{Z}{\|}}{C}—O—R \qquad (IV)$$

wherein G and Z are as defined for formula I and R is phenyl, alkyl or substituted phenyl, with an amine of formula V

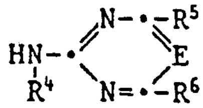

(V)

wherein E, $R^4$, $R^5$ and $R^6$ are as defined for formula I, and optionally converting the resulting compound of formula I into a salt.

14. A process for the preparation of compounds of formula I according to claim 1, which process comprises reacting a sulfonyl isocyanate or sulfonyl isothiocyanate of formula VI

$$G—SO_2—N=C=Z \quad (VI)$$

wherein G and Z are as defined for formula I, with an aminopyrimidine or aminotriazine of formula V according to claim 13, and optionally converting the resulting compound of formula I into a salt.

15. A process for the preparation of addition salts of formula I according to any one of claims 12 to 14, which process comprises reacting a sulfonylurea of formula I with an amine, an alkali metal hydroxide or an alkaline earth metal hydroxide or with a quaternary ammonium base.

16. A method of controlling undesired plant growth, which method comprises applying to the plants or to the locus thereof an effective amount of a compound of formula I according to claim 1, or of a composition containing such a compound

17. A method of inhibiting plant growth, which method comprises applying to the plants or to the locus thereof an effective amount of a compound of formula I according to claim 1, or of a composition containing such a compound.

18. A method of influencing plant growth in order to increase yield, which method comprises applying to the plants or to the locus thereof an effective amount of a compound of formula I according to claim 1, or of a composition containing such a compound.

19. A method according to claim 16 of selectively controlling weeds pre- or post-emergence in crops of useful plants.

20. A method according to claim 17 of inhibiting plant growth beyond the 2-leaf stage, which method comprises applying the active ingredients pre-emergence.

21. A method according to claim 19, wherein the crops are rice crops.